# EUROPÄISCHE PATENTSCHRIFT

(11) **EP 2 780 313 B1**
(45) Veröffentlichungstag und Bekanntmachung des Hinweises auf die Patenterteilung: **24.02.2016**
(21) Anmeldenummer: 12780101.7
(22) Anmeldetag: 24.10.2012
(51) Int. Cl.: C07C 41/42, C07C 41/44, C07C 43/13, C07C 29/80

(54) **DESTILLATIVES VERFAHREN ZUR GEWINNUNG VON DI-TRIMETHYLOLPROPAN**
METHOD FOR RECOVERING DI-TRIMETHYLOLPROPANE BY DISTILLATION
PROCÉDÉ D'OBTENTION DE DI-TRIMÉTHYLOLPROPANE PAR DISTILLATION

(30) Priorität: 19.11.2011 DE 102011118953
(43) Veröffentlichungstag der Anmeldung: 24.09.2014
(73) Patentinhaber: OXEA GmbH, 46147 Oberhausen (DE)
(72) Erfinder: FREY, Guido, D., 64560 Riedstadt (DE); NOWOTNY, Norman, 45276 Essen (DE); SCHALAPSKI, Kurt, 46147 Oberhausen (DE); STRUTZ, Heinz, 47445 Moers (DE)
(86) Internationale Anmeldenummer: PCT/EP2012/004439
(87) Internationale Veröffentlichungsnummer: WO 2013/072007

(56) Entgegenhaltungen:
- EP-A1- 0 625 503
- EP-A1- 1 848 679
- EP-A2- 1 178 030
- WO-A1-92/05134
- DE-A1-102008 038 021
- JP-A- 2000 302 725
- JP-A- 2003 267 904
- JP-A- 2007 197 466
- US-A- 3 962 347
- US-A1- 2003 139 631

## Beschreibung

Die vorliegende Erfindung betrifft ein destillatives Verfahren zur Gewinnung von Di-Trimethylolpropan.

Trimethylolpropan ist ein dreiwertiger Alkohol, der für die Herstellung von Lacken, Polyurethanen und Polyestern, wie beispielsweise von Alkydharzen, von Bedeutung ist. Industriell wird Trimethylolpropan durch Kondensationsreaktion von n-Butyraldehyd mit Formaldehyd nach verschiedenen Varianten hergestellt.

Bei dem sogenannten Hydrierverfahren addiert man zumindest zwei Mole Formaldehyd mit einem Mol n-Butyraldehyd in Gegenwart einer katalytischen Menge eines tertiären Amins über die Zwischenstufe Monomethylolbutyraldehyd zunächst zum Dimethylolbutyraldehyd, der anschließend in einem Hydrierschritt zum Trimethylolpropan umgesetzt wird. Nach dem in WO98/28253 A1 beschriebenen Verfahren wird Formaldehyd mit bis zu einem achtfachen molaren Überschuss eingesetzt. Das erhaltene Reaktionsgemisch aus dem Aldoladditionsschritt wird entweder destillativ oder durch Phasentrennung aufgearbeitet. Bei der destillativen Aufarbeitung werden nicht umgesetzte oder teilumgesetzte Ausgangsverbindungen als flüchtige Komponenten abgezogen und in die Reaktionsstufe zurückgeführt, während das Sumpfprodukt weiter umgesetzt wird. Wird anstelle der destillativen Aufarbeitung das Reaktionsgemisch in einem Phasentrenner in die wässrige und organische Phase geschieden, wird die organische Phase in die Aldoladdition zurückgefahren und die wässrige Phase weiter verarbeitet. Es folgt eine katalytische und/oder thermische Behandlung, um Monomethylolbutyraldehyd in Dimethylolbutyraldehyd zu überführen. Hierbei gebildete Nebenprodukte werden destillativ abgetrennt und das Sumpfprodukt dieser Destillation wird anschließend katalytisch hydriert, um Trimethylolpropan zu gewinnen. Das erhaltene rohe Trimethylolpropan wird anschließend einer Reindestillation unterzogen. Nach Leicht- und Mittelsiederabtrennung erhält man gereinigtes Trimethylolpropan als Zwischenfraktion, während höhersiedende Konderisationsprodukte, in denen Trimethylolpropanäquivalente gebunden sind, als Nachlauf- oder Sumpffraktion anfallen.

Neben dem Hydrierverfahren wird Trimethylolpropan technisch auch über die sogenannte Cannizzaro-Reaktion hergestellt. In einer ersten Reaktionsstufe lässt man dabei n-Butyraldehyd und Formaldehyd unter Zugabe von stöchiometrischen Mengen einer Base zum Dimethylolbutyraldehyd reagieren, das anschließend mit überschüssigem Formaldehyd zum Trimethylolpropan reduziert wird, während gleichzeitig ein Äquivalent Formiat gebildet wird. Üblicherweise wird als Base eine wässrige Lösung einer Alkalimetall- oder Erdalkalimetallverbindung verwendet, beispielsweise Natrium-, Kalium- oder Calciumhydroxid. Da ein Äquivalent Alkalimetall- oder Erdalkalimetallformiat bei dem Cannizzaro-Verfahren als Koppelprodukt anfällt, hängt die Wirtschaftlichkeit dieser Verfahrensvariante auch von den Vermarktungschancen dieses Koppelprodukts ab. Die Aufarbeitung der erhaltenen, wässrigen Reaktionslösung, die Trimethylolpropan, Alkalimetall- oder Erdalkalimetallformiat und überschüssige Base enthält, erfolgt im Allgemeinen durch Extraktion. Nach Neutralisation der überschüssigen Base wird die wässrige Lösung mit einem organischen Lösungsmittel, beispielsweise mit Ethylacetat extrahiert. Die organische Phase wird von der wässrigen Phase, die die Alkalimetall- oder Erdalkalimetallformiate gelöst enthält, getrennt und nach Entfernung des Extraktionsmittels wird Trimethylolpropan durch Destillation gewonnen. Das erhaltene Trimethylolpropan kann weiteren Reinigungsverfahren unterzogen werden. Nach US 5,603,835 wird zunächst aus erhaltenem Trimethylolpropan eine wässrige Lösung hergestellt, die nochmals mit einem organischen Lösungsmittel, beispielsweise mit Methyl-tertiär-butylether, extrahiert wird. Aus der erhaltenen wässrigen Lösung wird Trimethylolpropan mit einer verbesserten Farbzahl von weniger als 100 APHA Einheiten gewonnen.

Gemäß dem aus US 5,948,943 bekannten Verfahren wird die nach der Cannizzaro-Reaktion erhaltene wässrige, rohe Reaktionslösung bei einer solchen Temperatur mit einem geeigneten organischen Lösungsmittel behandelt, dass nur eine flüssige Phase das Extraktionsgefäß verlässt. Bei der anschließenden Abkühlung außerhalb des Extraktionsgefäßes trennt sich die wässrige von der organischen Phase und aus der wässrigen Phase kann Trimethylolpropan mit einer Farbzahl von weniger als 100 APHA isoliert werden.

Es ist ebenfalls bekannt, die Cannizzaro-Reaktion mit einer organischen Base, beispielsweise mit einem tertiären Amin, durchzuführen. Nach der aus WO97/17313 A1 bekannten Arbeitsweise wird Formaldehyd mit n-Butyraldehyd in Gegenwart von stöchiometrischen Mengen eines tertiären Amins umgesetzt, wobei ein Äquivalent des Ammoniumformiats entsteht. Anschließend wird aus dem Rohgemisch Wasser, überschüssiges tertiäres Amin und überschüssiger Formaldehyd abgetrennt und das verbleibende Gemisch erhitzt. Dabei wird das Ammoniumformiat in das tertiäre Amin und Ameisensäure gespalten, wobei das tertiäre Amin und weitere flüchtige Bestandteile abgetrennt werden und es zur Bildung von Trimethylolpropanformiat kommt. Das abgetrennte tertiäre Amin wird entweder in die Cannizzaro-Stufe zurückgeführt oder als Katalysator für die Umesterung des Trimethylolpropanformiats in einer nachgeschalteten Reaktion mit einem zugesetzten niedrigen aliphatischen Alkohol verwendet. Das dabei freigesetzte Trimethylolpropan wird anschließend aus dem Rohprodukt isoliert.
Unabhängig davon, ob die Herstellung von Trimethylolpropan nach dem Hydrierverfahren unter Verwendung katalytischer Mengen eines tertiären Amins, nach dem Cannizzaro-Verfahren mit molaren Mengen eines tertiären Amins und nachfolgender Umesterung des gebildeten Trimethylolpropanformiats oder nach dem Cannizzaro-Verfahren mit molaren Mengen von Alkalimetall- oder Erdalkalimetallhydroxiden und ihrer extraktiven Abtrennung erfolgt, wird das erhaltene rohe Trimethylolpropan einer ein- oder mehrfachen destillativen Reinigung unterzogen, die aufgrund des hohen Siedepunkts im Unterdruck erfolgt. Nach DE 100 58 303 A1 wird die destillative Aufarbeitung des Trimethylolpropans mit einer Ionenaustauscherbehandlung kombiniert, wobei entweder der Aldolisierungsaustrag oder der Hydrieraustrag vor der destillativen Aufarbeitung mit einem stark basischen Ionenaustauscher in Kontakt gebracht wird.

Bei der destillativen Reinigung fallen schwersiedende Fraktionen mit einem im Vergleich zum Trimethylolpropan höheren Siedepunkt oder Rückstände an, in denen Derivate des Trimethylolpropans vorliegen und aus diesem durch Reaktion mit beispielsweise Methanol, Formaldehyd oder auch mit einem weiteren Molekül Trimethylolpropan in den vorgeschalteten Reaktionen entstanden sind. Unter diesen Derivaten sind besonders formaldehydhaltige Acetale vertreten, die durch das Strukturelement -O-CH₂-O- charakterisiert werden und auch als Formale aufgefasst werden können. Unter den Formalen können folgende lineare und zyklische Formale des Trimethylolpropans strukturell beschrieben werden:
Monozyklisches Formal des Trimethylolpropans: 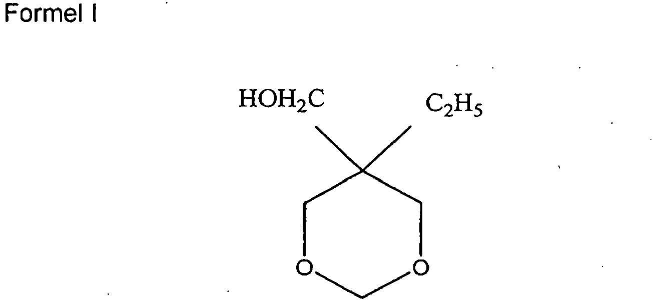
Lineares bis-Trimethylolpropan-Formal:

   Formel II [C₂H₅C(CH₂OH)₂CH₂O]₂CH₂
Methyl-(monolineares) Formal des Trimethylolpropans:

   Formel III C₂H₅C(CH₂OH)₂CH₂OCH₂OCH₃
Methyl-(bis-lineares) Formal des Trimethylolpropans:

   Formel IV C₂H₅C(CH₂OH)₂CH₂OCH₂OCH₂OCH₃

Hierbei siedet das monozyklische Formal des Trimethylolpropans (I) bei einer niedrigeren Temperatur als Trimethylolpropan selbst. Die vom Methanol abgeleiteten Formale (III) und (IV) haben einen mit Trimethylolpropan vergleichbaren Siedepunkt während das lineare bis-Trimethylolpropan-Formal (Formel II) als schwersiedende Komponente vorliegt. Darüber hinaus sind noch weitere lineare und zyklische sauerstoffhaltige Verbindungen, wie das zyklische Formal des Di-Trimethylolpropans 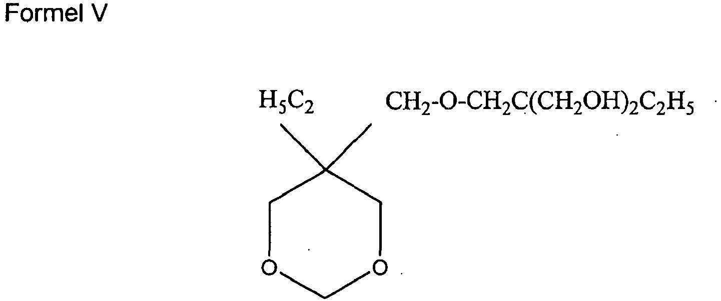 in den Destillationsrückständen zugegen.

Ebenfalls sind in den schwersiedenden Fraktionen und Rückständen der destillativen Aufarbeitung des rohen Trimethylolpropans noch substantielle Mengen an Di-Trimethylolpropan [C₂H₅C(CH₂OH)₂-CH₂-]₂-O und Trimethylolpropan selbst enthalten. Darüber hinaus sind leichtsiedende Komponenten, wie Methanol, 2-Methylbutanol oder 2-Ethyl-2-Methyl-1,3-Propandiol in geringen Mengen anwesend.

Da in den schwersiedenden Fraktionen und Rückständen der destillativen Aufarbeitung des Trimethylolpropans erhebliche Mengen an Derivaten vorliegen, in denen Äquivalente des Trimethylolpropans chemisch gebunden sind, werden eine Reihe von Verfahren vorgeschlagen, um insbesondere formaldehydhaltige Acetale zu spalten und Trimethylolpropan freizusetzen, um auf diese Weise die Ausbeute des gesamten Trimethylolpropanherstellprozesses zu verbessern. Nach WO 2004/013074 A1 werden die bei der Trimethylolpropanherstellung erhaltenen schwersiedenden Fraktionen sowie Destillationsrückstände mit Säure behandelt, wobei der Wassergehalt im Reaktionsansatz 20-90 Gew.-% betragen soll. Aus dem säurebehandelten Produkt kann entweder Trimethylolpropan destillativ gewonnen werden oder das behandelte Produkt kann in die Hydrierstufe des Dimethylolbutyraldehyds zum Trimethylolpropan zurückgeführt werden.

Die hydrierende Spaltung von linearen oder zyklischen Acetalen in wässrigen Lösungen in Gegenwart eines heterogenen Hydrierkatalysators zu dem gewünschten mehrwertigen Alkohol ist aus DE 198 40 276 A1 bekannt. Das Verfahren erfordert Hydriertemperaturen oberhalb von 160°C, um den schädlichen Einfluss von Formiaten, die besonders bei dem Arbeiten nach dem Cannizzaro-Verfahren noch zugegen sein können, auf die Hydrierleistung des Katalysators zurückzudrängen. Die hydrierende, katalytische Spaltung kann ebenfalls in Gegenwart einer Säure, beispielsweise in Gegenwart einer niederen Carbonsäure oder sauer reagierender Feststoffe durchgeführt werden. Nach der WO 97/01523 kann die Hydriertemperatur zwar erniedrigt werden, doch ist dann ein hohes Gewichtsverhältnis von dem katalytisch wirksamen Metall zu dem zyklischen Formal einzustellen, um eine vertretbare Spaltrate zu erzielen.

Die schwersiedenden Fraktionen und die Rückstände der destillativen Aufarbeitung der Trimethylolpropanherstellung enthalten neben den zuvor erwähnten formaldehydhaltigen Acetalen auch bedeutende Mengen an Di-Trimethylolpropan, das ebenfalls als wertvolles Ausgangsmaterial zur Herstellung von Alkydharzen, Weichmachern und Schmiermitteln technische Bedeutung besitzt. Aus dem Stand der Technik sind Verfahren bekannt, Di-Trimethylolpropan aus diesen Rückständen zu gewinnen und so erhaltenes Produkt bei Bedarf weiter zu reinigen.

Nach DE 2058518 A1 wird der Di-Trimethylolpropan haltige Destillationsrückstand einer Wasserdampfdestillation mit überhitztem Wasserdampf unter reduziertem Druck unterzogen. Aus dem erhaltenen wässrigen Destillat wird nach Wasserabtrennung Di-Trimethylolpropan erhalten, das im Bedarfsfalle aus einem organischen Lösungsmittel, beispielsweise Aceton, umkristallisiert werden kann.

Der Stand der Technik weist mehrfach auf Schwierigkeiten hin, die mit der destillativen Reinigung von Di-Trimethylolpropan aus den Nebenströmen der Trimethylolpropanherstellung verbunden sind. Zum einen kann der Gehalt an Di-Trimethylolpropan im Destillationsrückstand erheblich schwanken. Ferner enthält der Destillationsrückstand ebenfalls hochsiedendes lineares bis-Trimethylolpropan-Formal (Formel II), das nur sehr schwierig abgetrennt werden kann. Auch erfordert der hohe Siedepunkt des Di-Trimethylolpropans die Anwendung sehr niedriger Destillationsdrücke und hoher Destillationstemperaturen, so dass bei der hohen thermischen Belastung mit Zersetzungsreaktionen zu rechnen ist.

Nach dem aus EP 2204356 A1 bekannten Verfahren umgeht man diese Schwierigkeiten dadurch, indem man den Gehalt an Di-Trimethylolpropan gezielt in dem Reaktionsgemisch erhöht. Zunächst werden pro Mol n-Butyraldehyd 2,0 bis 3,5 mol Formaldehyd in Gegenwart einer Base umgesetzt. Gebildetes 2-Ethylacrolein wird abdestilliert und zusammen mit weiterem Formaldehyd dem trimethylolpropanhaltigen Destillationsrückstand wieder zugesetzt, wobei es zur Bildung von Di-Trimethylolpropan und Ameisensäure kommt. Nach Neutralisation wird der Ansatz von Leichtsiedern befreit und entweder durch Destillation oder Kristallisation aufgearbeitet. Gemäß EP 1178030 A2 wird der Rückstand aus der Trimethylolpropanherstellung nach Extraktion und Trimethylolpropanabtrennung mit einer Säure behandelt, um Formale zu spalten. Anschließend wird Di-Trimethylolpropan an einem Fallfilmverdampfer abdestilliert und durch Kristallisation gereinigt.

Ebenfalls wird die direkte Aufarbeitung des Destillationsrückstands aus der Trimethylolpropanherstellung durch Umkristallisation zur Gewinnung von Di-Trimethylolpropan beschrieben. DE 2358297 A1 behandelt die einfache Kristallisation einer wässrigen Lösung des Destillationsrückstands, wobei in der wässrigen Lösung die Salzkonzentration auf ein besonderes Verhältnis eingestellt wird, um das Ausfällen von Di-Trimethylolpropan in ausreichender Reinheit zu ermöglichen. Wenn Trimethylolpropan nach dem Cannizzaro-Verfahren hergestellt wird, kann der Salzgehalt, beispielsweise der Alkalimetallformiatgehalt in dem Destillationsrückstand bereits genügend hoch sein, um das Ausfallen von Di-Trimethylolpropankristallen in befriedigenderweise nach Auflösung in Wasser sicherzustellen. Gegebenenfalls ist der wässrigen Lösung ein weiteres Salz zuzusetzen, beispielsweise ein Alkalimetallsalz.

US 2004/0254405 A1 offenbart ein Verfahren zur Umkristallisation des Destillationsrückstands unter Verwendung organischer Lösungsmittel, beispielsweise Aceton oder Methylethylketon, wobei die Kristallisationstemperatur, die Lösungsmittelmenge und der Gehalt an Di-Trimethylolpropan im Destillationsrückstand in besonderer Weise einzuhalten sind. Die Verwendung eines Gemisches aus einem geeigneten Lösungsmittel und Wasser für die Isolierung von Di-Trimethylolpropan aus den Destillationsrückständen der Trimethylolpropanherstellung wird in DE 10 2008 038 021 A1 beschrieben. Man erhält zunächst eine organische Lösungsmittelphase und einen viskosen Rückstand, trennt die Phasen und extrahiert die organische Lösungsmittelphase mit Wasser. Die Wasserphase wird abgetrennt und enthaltene Lösungsmittelreste werden entfernt. Aus der verbleibenden Wasserphase wird Di-Trimethylolpropan kristallisiert.

In der Deutschen Patentanmeldung 10 2010 033 844 A1 wird ebenfalls ein Verfahren zur Gewinnung von Di-Trimethylolpropan aus den Nebenströmen der Trimethylolpropanherstellung behandelt. Dabei werden die anfallenden schwersiedenden Fraktionen und Rückstände in Wasser aufgelöst und die wässrige Lösung wird in Gegenwart einer aciden Verbindung unter Spaltung von formaldehydhaltigen Acetalen katalytisch hydriert. Das wässrige Hydriergut wird nach Feststoffabtrennung anschließend sowohl mit basischen als auch mit sauren Ionenaustauschern in Kontakt gebracht. Aus dem wässrigen Eluat wird ein mit Trimethylolpropan angereicherter Produktstrom abdestilliert und Di-Trimethylolpropan verbleibt als Destillationsrückstand. Damit Di-Trimethylolpropan in ausreichender Qualität im Destillationsrückstand anfällt, ist nach dem Prozess gemäß der Deutschen Patentanmeldung 10 2010 033 844 A1 die Behandlung des wässrigen Hydrierguts sowohl mit basischen als auch mit sauren Ionenaustauschern zwingend notwendig.

Schließlich werden im Stand der Technik auch Verfahren für die gezielte Synthese von Di-Trimethylolpropan beschrieben. Nach EP 0799815 A1 wird Trimethylolpropan direkt mit 2-Ethylacrolein und Formaldehyd in Gegenwart basischer Katalysatoren umgesetzt. Nach Abtrennung flüchtiger Anteile wird gereinigtes Di-Trimethylolpropan durch Umkristallisation aus Wasser erhalten. Gemäß WO 92/05134 A1 wird bereits isoliertes Trimethylolpropan in Gegenwart einer sauer reagierenden Verbindung erhitzt, wobei es unter Veretherung zur Bildung von Di-Trimethylolpropan kommt. Um die Bildung von höheren Kondensationsprodukten des Trimethylolpropans zu vermeiden, wird Trimethylolpropan bis maximal 30% umgesetzt. Aus dem erhaltenen Reaktionsgemisch werden die flüchtigen Anteile destillativ abgetrennt. Auch Di-Trimethylolpropan kann zunächst abdestilliert werden und weiter durch Umkristallisation gereinigt werden. J. Org. Chem., Vol. 37, No. 13, 1972, Seiten 2197-2201 offenbart ebenfalls die direkte Herstellung von Di-Trimethylolpropan. Zunächst wird Trimethylolpropan mit Aceton in das Ketal überführt, um zwei Hydroxylgruppen zu schützen. Anschließend wird die noch freie Hydroxylgruppe in die Tosylat- oder Mesylatgruppe überführt, die in Gegenwart von Basen unter Etherbildung abgespalten wird. Durch Säurezugabe wird die Ketalschutzgruppe entfernt und Di-Trimethylolpropan freigesetzt, das aus Wasser umkristallisiert wird. Dieses in der wissenschaftlichen Literatur beschriebene Verfahren ist jedoch aufwendig und erfordert spezielle Chemikalien durch das Einführen und Abspalten von Schutzgruppen.

Die bekannten Verfahren zur Gewinnung von Di-Trimethylolpropan aus schwersiedenden Fraktionen und Rückständen, die einen höheren Siedepunkt als Trimethylolpropan aufweisen und die bei der destillativen Aufarbeitung im Rahmen der Trimethylolpropanherstellung oder bei der gezielten Synthese von Di-Trimethylolpropan anfallen, erfordern entweder aufwendige Umkristallisationsschritte oder eine aufwendige Wasserdampfdestillation mit der nachfolgenden Wasserentfernung aus dem Wasserdampfdestillat. Der Stand der Technik erwähnt zwar die destillative Abtrennung von Di-Trimethylolpropan unter Verwendung eines Fallfilm-Verdampfers, doch wird zur weiteren Reinigung des Destillats eine Umkristallisation empfohlen.

Auch wird bei Verfahren, bei denen Di-Trimethylolpropan als Destillationsrückstand anfällt, Di-Trimethylolpropan nicht immer in ausreichender Qualität erhalten, um es in möglichst vielen technischen Anwendungen einzusetzen. Zudem ist vor der Destillationsstufe eine Reinigung mit Ionenaustauschern notwendig, um den Gehalt an Verunreinigungen im Destillationsrückstand möglichst gering zu halten.

Es besteht daher Bedarf an einem Verfahren, Di-Trimethylolpropan möglichst einfach und in hoher Reinheit zu erhalten. Insbesondere soll der Gehalt an linearem bis-Trimethylolpropan-Formal (Formel II) und weiteren hochsiedenden Verbindungen, die ein dem Di-Trimethylolpropan ähnliches Siedeverhalten zeigen, bis auf eine akzeptable Grenze reduziert werden. Dabei sollen aufwendige Umkristallisationsschritte vermieden werden. Auch soll das Verfahren allgemein einzusetzen sein und unabhängig davon, ob Di-Trimethylolpropan bei der Herstellung von Trimethylolpropan als Nebenprodukt anfällt, ob die Nebenströme bei der Trimethylolpropanherstellung weiteren Prozessschritten, wie einer Säurebehandlung, einer Ionenaustauscherbehandlung oder einer katalytischen Hydrierung in Gegenwart einer Säure unterworfen werden, oder ob Di-Trimethylolpropan durch eine gezielte Synthesen hergestellt wird.

Die vorliegende Erfindung betrifft daher ein destillatives Verfahren zur Gewinnung von Di-Trimethylolpropan aus Di-Trimethylolpropan enthaltenden Lösungen, dadurch gekennzeichnet, dass man:
(a) die Di-Trimethylolpropan enthaltende Lösung in einer ersten Destillationseinrichtung in eine erste Kopffraktion enthaltend leichtsiedende, niedriger als Di-Trimethylolpropan siedende Verbindungen und in eine Sumpffraktion auftrennt;
(b) die nach Schritt a) erhaltene Sumpffraktion auf eine zweite Destillationseinrichtung mit mindestens 5 theoretischen Böden, die als Dünnschichtverdampfer mit Kolonnenaufsatz ausgestaltet ist, gibt und eine zweite Kopffraktion enthaltend zwischensiedende, niedriger als Di-Trimethylolpropan siedende Verbindungen abzieht und eine Sumpffraktion entnimmt; und
(c) die nach Schritt b) erhaltene Sumpffraktion auf eine dritte Destillationseinrichtung mit mindestens 4 theoretischen Böden, die als Dünnschichtverdampfer mit Kolonnenaufsatz ausgestaltet ist, gibt, in der Di-Trimethylolpropan als dritte Kopffraktion gewonnen wird und Hochsieder als Sumpffraktion entfernt werden.

Einsatzströme für das erfindungsgemäße Destillationsverfahren sind Di-Trimethylolpropan enthaltende Lösungen, die unabhängig von einem bestimmten Produktionsverfahren anfallen. Beispielsweise erhält man diese Lösungen bei der Herstellung von Trimethylolpropan, entweder nach dem Cannizzaro-Verfahren unter Verwendung von Alkali- oder Erdalkalimetallverbindungen oder nach dem Hydrierverfahren in Gegenwart katalytischer Mengen von Trialkylaminen oder nach dem Cannizzaro-Verfahren unter Einsatz stöchiometrischer Mengen an Trialkylaminen. Insbesondere fallen sie bei der destillativen Aufarbeitung von Trimethylolpropan als schwersiedende Rückstände an. Häufig werden diese schwersiedenden Rückstände zur Spaltung der Formale nach den Formeln I bis V in Wasser aufgenommen und mit einer Säure behandelt oder in wässriger Lösung mit Wasserstoff in Gegenwart einer aciden Verbindung und eines Metallkatalysators hydriert.

Für das erfindungsgemäße Destillationsverfahren geeignete Lösungen erhält man ebenfalls bei Prozessen, in denen Di-Trimethylolpropan gezielt hergestellt wird, beispielsweise durch Umsetzung von bereits isoliertem Trimethylolpropan mit 2-Ethylacrolein und Formaldehyd in Gegenwart eines basischen Katalysators oder durch Umsetzung von n-Butyraldehyd und Formaldehyd in Gegenwart eines basischen Katalysators unter solchen Bedingungen, dass zunächst 2-Ethylacrolein aus dem trimethylolpropanhaltigen Reaktionsgemisch abdestilliert wird und zusammen mit weiterem Formaldehyd erneut dem Reaktionsgemisch zugesetzt wird, um in der ersten Stufe gebildetes Trimethylolpropan in Di-Trimethylolpropan zu überführen. Auch durch Veretherung einer Hydroxylgruppe des Trimethylolpropans, gegebenenfalls nach Einführen einer Schutzgruppe für die beiden übrigen Hydroxylgruppen, kann man geeignete Di-Trimethylolpropan enthaltende Lösungen gewinnen.

Ebenfalls kann in dem erfindungsgemäßen Destillationsverfahren Di-Trimethylolpropän eingesetzt werden, das bereits durch eine vorherige Umkristallisation gereinigt wurde oder durch die Behandlung mit einem Ionenaustauscher vorgereinigt wurde. Für die Ionenaustauscherbehandlung eignen sich beispielsweise übliche kommerziell verfügbare saure und basische Ionenaustauscher. Die Di-Trimethylolpropan enthaltende Lösung kann entweder nur mit einem basischen oder sauren Ionenaustauscher oder in beliebiger Kombination in Kontakt gebracht werden.

Das erfindungsgemäße Verfahren eignet sich besonders bei der destillativen Aufarbeitung von Lösungen, die Di-Trimethylolpropan in einem polaren Lösungsmittel gelöst enthalten, insbesondere bei der destillativen Aufarbeitung von wässrigen Lösungen oder Lösungen in einem niederen C₁-C₅-aliphatischen Alkohol oder einem C₂-C₁₀-Dialkylether, wie Methanol, Ethanol, Propanol oder Diethylether. Solche alkoholischen und insbesondere wässrigen Lösungen fallen insbesondere bei der sauren Behandlung von Rückständen aus der Trimethylolpropanherstellung an, die zusätzlich noch unter Wasserstoff in Gegenwart eines Hydrierkatalysators erfolgen kann.

Diese Rückstände aus der Trimethylolpropanherstellung enthalten als Hauptkomponenten noch physikalisch eingemischtes Trimethylolpropan, im Allgemeinen in einem Bereich von 5 bis 30 Gew.-%, Di-Trimethylolpropan, im Allgemeinen in einem Bereich von 10 bis 35 Gew.-% sowie das lineare bis-Trimethylolpropan-Formal (Formel II) in einem Bereich von 25 bis 70 Gew.-%, bezogen auf die gesamte Einsatzmasse. Weitere identifizierte Produkte sind 2-Ethyl-2-Methyl-1,3-Propandiol und das monozyklische Formal des Trimethylolpropans (Formel I), die aufgrund ihres vergleichsweise geringen Siedepunktes nur noch in geringen Mengen bis üblicherweise bis zu 3 Gew.-% anwesend sind. Neben dem Methyl-(monolinearen) Formal des Trimethylolpropans (Formel III), dem Methyl-(bis-linearen) Formal des Trimethylolpropans (Formel IV) sowie dem zyklischen Formal des Di-Trimethylolpropans (Formel V) oder den in einer gegebenenfalls vorgeschalteten Hydrierung aus den Methylethern freigesetzten Formalen des Trimethylolpropans wie:

C₂H₅C(CH₂OH)₂CH₂OCH₂OH Formel VI, CAS 482619-74-9

C₂H₅C(CH₂OH) (CH₂OCH₂OH)₂ Formel VII

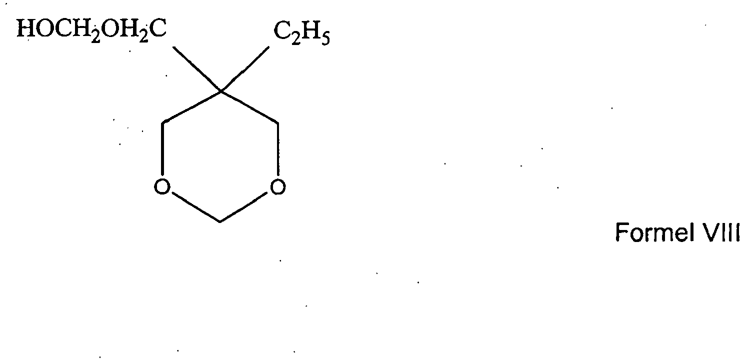

sind noch eine Reihe weiterer linearer oder zyklischer Formale des Di-Trimethylolpropans zugegen, wie:

C₂H₅C(CH₂OH)₂CH₂OCH₂C(C₂H₅)(CH₂OH)(CH₂OCH₂OH) Formel IX, CAS 97416-99-4

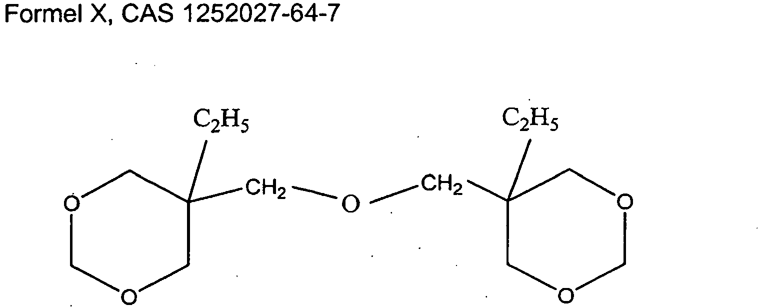

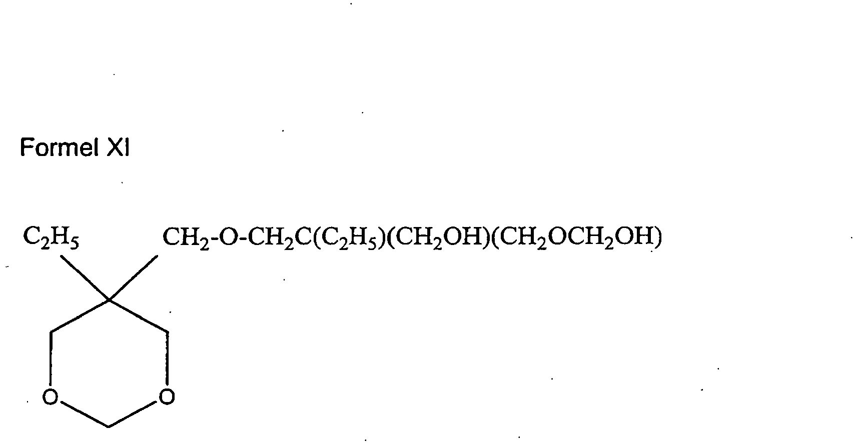

sowie das monozyklische Formal aus dem linearen bis-Trimethylolpropan-Formal (Formel II)

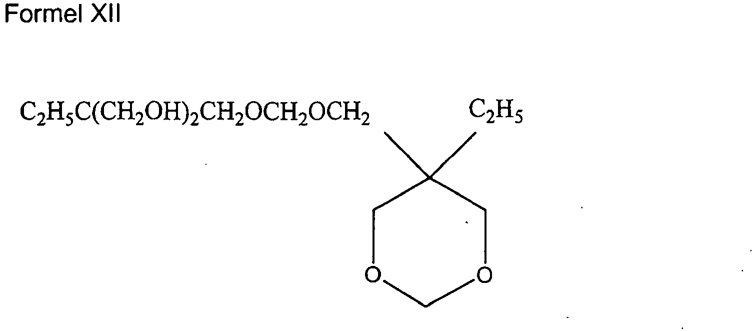

Diese Rückstände aus der Trimethylolpropanherstellung werden mit einem polaren Lösungsmittel, vorzugsweise mit Wasser, unter Bildung einer Lösung versetzt. Im Allgemeinen stellt man eine Lösung mit einem Gehalt an organischen Komponenten ohne Berücksichtigung des polaren Lösungsmittels von 30 bis 90 Gew.-%, vorzugsweise von 50 bis 80 Gew.-% her, bezogen auf die Gesamtmasse. Geringere Gehalte an organischen Komponenten sind aufgrund des hohen Lösungsmittelanteils nicht zweckmäßig, bei zu hohen Gehalten ist besonders bei Raumtemperatur mit Inhomogenitäten in der Lösung oder mit dem Ausfallen von Feststoffen zu rechnen. Zweckmäßigerweise wird die Lösung bei einer Temperatur von über 50°C hergestellt. Ein Temperaturbereich für die Lösung, insbesondere für die wässrige Lösung, von 60°C bis 80°C ist vorzugsweise einzustellen.

Gegebenenfalls kann die erhaltene Lösung bei erhöhter Temperatur und erhöhtem Druck mit Wasserstoff in Gegenwart eines Katalysators und einer aciden Verbindung behandelt werden. Durch die Hydrierung im sauren Medium werden die vorhandenen Formale gespalten und freigesetztes Formaldehyd in Methanol überführt. Gegebenenfalls kann die erhaltene Lösung noch mit einem Ionenaustauscher oder in Kombination mit sauren und basischen Ionenaustauschern in Kontakt gebracht werden.

Unabhängig von dem Herstellprozess wird die Di-Trimethylolpropan enthaltende Lösung gemäß dem erfindungsgemäßen Verfahren in einer dreistufigen Destillationseinrichtung aufgearbeitet. Unter der Di-Trimethylolpropan enthaltenden Lösung wird auch eine Lösung verstanden, die bereits aufgereinigtes Di-Trimethylolpropan, beispielsweise aus einem Umkristallisationsprozess, enthält, in der der Gehalt an Verunreinigungen mit einem ähnlichen Siedepunkt wie Di-Trimethylolpropan schon abgereichert ist. Eine solche Verunreinigung mit einem ähnlichen Siedepunkt wie Di-Trimethylolpropan ist beispielsweise das lineare bis-Trimethylolpropan-Formal (Formel II), das monozyklische Formal aus dem linearen bis-Trimethylolpropan-Formal (Formel XII), das monolineare Formal des Di-Trimethylolpropans (Formel IX) oder das monolineare Formal des zyklischen Formals des Di-Trimethylolpropans (Formel XI). Als Lösungsmittel für bereits aufgereinigtes Di-Trimethylolpropan eignen sich Wasser sowie niedere C₁-C₅-aliphatische Alkohole, wie Methanol, Ethanol oder Propanol. Auch bereits isoliertes, festes Di-Trimethylolpropan wird nach dem Aufschmelzen als Di-Trimethylolpropan enthaltende Lösung im Sinne der vorliegenden Erfindung angesehen.

Die Di-Trimethylolpropan enthaltende Lösung wird in einer dreistufigen Destillationseinrichtung aufgearbeitet. Zunächst werden in einer ersten Destillationseinrichtung Leichtsieder, als erste Kopffraktion abgetrennt. Für die Leichtsiederabtrennung eignen sich übliche Destillationseinrichtungen, wie eine Destillationskolonne, die beispielsweise 2 bis 40 theoretische Böden aufweist, mit Sumpfblase, ein Dünnschichtverdampfer, ein Kurzwegverdampfer oder ein Abdampfbehälter, die üblicherweise bei Sumpftemperaturen von 100 bis 180°C und bei Normaldruck oder zweckmäßigerweise im Unterdruck bis zu 70 hPa betrieben werden. Der Zulauf zur ersten Destillationseinrichtung kann bei Raumtemperatur aufgegeben werden, vorteilhafterweise besitzt der Zulauf jedoch eine Temperatur von 50 bis 130°C, insbesondere 80 bis 120°C. Eine erhöhte Temperatur ist dann erforderlich, wenn eine Di-Trimethylolpropanschmelze aufgegeben wird. Durch die Aufgabe des Zulaufs, der bereits eine erhöhte Temperatur aufweist, können abzutrennende Leichtsieder schlagartig verdampfen und über die erste Kopffraktion abgeführt werden.

Wird eine Lösung von Di-Trimethylolpropan in einem polaren Lösungsmittel eingesetzt, dient die erste Destillationseinrichtung zur Abtrennung des polaren Lösungsmittels und von weiteren Leichtsiedern, beispielsweise Methanol, 2-Methylbutanol, 2-Ethyl-2-Methyl-1,3-propandiol oder leicht flüchtigen Formalen. Die erste Destillationseinrichtung wird dabei so betrieben, dass der Gehalt an dem polaren Lösungsmittel in der Di-Trimethylolpropan enthaltenden Sumpffraktion bis maximal 5000 Gew.-ppm, vorzugsweise bis 1000 Gew.-ppm und insbesondere bis 500 Gew.-ppm, bezogen auf die Masse der Sumpffraktion, liegt. Das Einhalten einer maximalen Grenze für den Lösungsmittelgehalt in der Sumpffraktion wirkt sich vorteilhaft auf die nachfolgende destillative Reinigung aus. Als polares Lösungsmittel eignet sich ein niederer C₁-C₅-aliphatischer Alkohol oder C₂-C₁₀-Di-Alkylether, wie Methanol, Ethanol, Propanol oder Diethylether oder insbesondere Wasser.

Diese Sumpffraktion aus der ersten Destillationseinrichtung wird abgeführt und auf eine zweite Destillationseinrichtung gegeben.

In der zweiten Destillationseinrichtung gewinnt man als zweite Kopffraktion einen zwischensiedenden Produktstrom, in dem Verbindungen vorliegen, die einen höheren Siedepunkt als die in der ersten Kopffraktion abgetrennten Leichtsieder aber einen niedrigeren Siedepunkt als Di-Trimethylolpropan besitzen. Wird ein Gemisch enthaltend Trimethylolpropan und Di-Trimethylolpropan aufgearbeitet, enthält die zweite Kopffraktion im Wesentlichen Trimethylolpropan und zudem noch Zwischenläufe und Reste des polaren Lösungsmittels und von Leichtsiedern. In der zwischensiedenden Kopffraktion sind beispielsweise noch das monolineare Formal des Trimethylolpropans (Formel VI), das bis-lineare Formal des Trimethylolpropans (Formel VII), das monolineare Formal des zyklischen Formals des Trimethylolpropans (Formel VIII) sowie das monozyklische Formal des Di-Trimethylolpropans (Formel V) und das bis-zyklische Formal des Di-Trimethylolpropans (Formel X) enthalten. Diese sauerstoffhaltigen Verbindungen kann man auch unter dem Zwischenlauf I zusammenfassen. Die zweite Kopffraktion kann dann zweckmäßigerweise in die Reinigungsstufe des Gesamtprozesses für die Herstellung von Trimethylolpropan zurückgeführt werden, vorteilhafterweise in die Reindestillation zur Gewinnung von Trimethylolpropan.

Die Abtrennung der zweiten Kopffraktion erfolgt an einer Destillationseinrichtung, die mindestens 5 theoretische Böden, vorzugsweise mindestens 8 theoretische Böden und insbesondere 8 bis 20 theoretische Böden aufweist. Ebenfalls ist in der zweiten Destillationseinrichtung die thermische Belastung möglichst gering zu halten und es ist bei möglichst geringen Verweilzeiten zu arbeiten. Dabei beträgt die Verweilzeit in der zweiten Destillationseinrichtung, also in der gesamten Destillationsapparatur im Allgemeinen von 2 bis 60, vorzugsweise von 10 bis 30 Sekunden. Als Anlagenschaltung verwendet man einen Dünnschichtverdampfer mit Kolonnenaufsatz, der die erforderliche Mindestanzahl an theoretischen Böden besitzt. Als Kolonnenaufsatz eignen sich konventionelle Füllkörper- oder Packungskolonnen, vorzugsweise Strukturpackungskolonnen. Solche Packungen sind kommerziell erhältlich, beispielsweise als Montz- oder Sulzerpackungen. Auch die in dem erfindungsgemäßen Verfahren einzusetzenden Dünnschichtverdampfer sind fachübliche Aggregate, die kommerziell zur Verfügung stehen. Nicht geeignet sind eine Sumpfblase mit Kolonnenaufsatz oder ein Kurzwegverdampfer, da bei dieser Anordnung die Verweilzeit in der Destillationseinrichtung entweder zu hoch ist oder die Trennleistung nicht ausreicht. Die zweite Destillationseinrichtung wird im Allgemeinen bei Sumpftemperaturen von 210 bis 280°C und bei einem Druck von 2 bis 10 hPa betrieben. Die Sumpffraktion aus der zweiten Destillationseinrichtung wird anschließend auf eine dritte Destillationseinrichtung gegeben.

Die dritte Destillationseinrichtung kann auch als Nachlaufabtrenneinrichtung angesehen werden und dient zur Gewinnung von Di-Trimethylolpropan in ausreichender Qualität. Di-Trimethylolpropan wird als dritte Kopffraktion abgetrennt und Hochsieder werden als Sumpffraktion aus der dritten Destillationseinrichtung entfernt. In den Hochsiedern sind beispielsweise das monozyklische Formal des linearen bis-Trimethylolpropan-Formals (Formel XII) und das zyklische Formal des Di-Trimethylolpropans (Formel XI) sowie das monolineare Formal des Di-Trimethylolpropans (Formel IX) enthalten. Diese sauerstoffhaltigen Verbindungen kann man auch unter dem Zwischenlauf II zusammenfassen. Um Di-Trimethylolpropan in ausreichender Qualität zu erhalten, hat die dritte Destillationseinrichtung mindestens 4 theoretische Böden und insbesondere 4 bis 20 theoretische Böden aufzuweisen. Ebenfalls ist auch in der dritten Destillationskolonne die thermische Belastung möglichst gering zu halten und es ist bei möglichst geringen Verweilzeiten zu arbeiten. Dabei beträgt die Verweilzeit der Kopffraktion in der dritten Destillationseinrichtung im Allgemeinen von 1 bis 30, vorzugsweise von 5 bis 20 Sekunden. Als Anlagenschaltung verwendet man ebenfalls einen Dünnschichtverdampfer mit Kolonnenaufsatz, der die erforderliche Mindestanzahl an theoretischen Böden besitzt. Als Kolonnenaufsatz eignen sich konventionelle Füllkörper- oder Packungskolonnen, vorzugsweise Strukturpackungskolonnen. Solche Packungen sind kommerziell erhältlich, beispielsweise als Montz- oder Sulzerpackungen. Auch die in dem erfindungsgemäßen Verfahren einzusetzenden Dünnschichtverdampfer sind fachübliche Aggregate, die kommerziell zur Verfügung stehen. Nicht geeignet sind eine Sumpfblase mit Kolonnenaufsatz oder ein Kurzwegverdampfer, da bei dieser Anordnung die Verweilzeit des Kopfproduktes in der Destillationseinrichtung entweder zu hoch ist oder die Trennleistung nicht ausreicht. Die dritte Destillationseinrichtung wird im Allgemeinen bei Sumpftemperaturen von 240 bis 280°C und bei einem Druck von 0,2 bis 5 hPa betrieben. Eine Sumpftemperatur von maximal 280°C sollte nicht überschritten werden, um eine zu starke Zersetzung von Di-Trimethylolpropan zu vermeiden.

Das über die Kopffraktion abgeführte Di-Trimethylolpropan fällt in einer für technische Anwendungen ausreichenden Qualität an und es können gaschromatographisch ermittelte Wertproduktgehalte von größer als 98 Gew.-% erhalten werden. Durch das erfindungsgemäße Destillationsverfahren können hochsiedende Verunreinigungen, die einen vergleichbar hohen Siedepunkt wie Di-Trimethylolpropan aufweisen, beispielsweise lineares bis-Trimethylolpropan-Formal (Formel II) oder Verbindungen, die höher als Trimethylolpropan aber vergleichbar mit Di-Trimethylolpropan sieden, wie die unter Zwischenlauf I zusammengefassten Verbindungen, wirksam abgetrennt werden. Auch können Sulfataschegehalte, bestimmt nach DIN 51575, modifiziert unter Zugabe von Schwefelsäure nach dem Abbrennen und vor dem Glühen der Probe, in dem destillativ gereinigten Di-Trimethylolpropan von unter 100 ppm und im Allgemeinen zwischen 15 und 80 ppm erreicht werden.

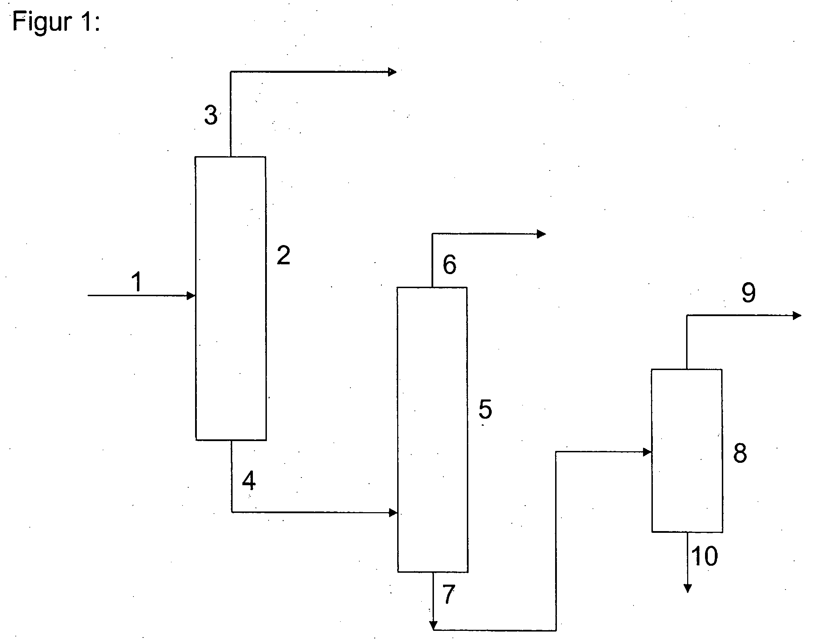

Figur 1 zeigt ein Prinzipschema für die destillative Behandlung der Di-Trimethyolpropan enthaltenden Lösung. Die über Leitung (1) herangeführte vorzugsweise erhitzte Lösung wird auf eine erste Destillationseinrichtung (2) gegeben, bei der am Kopf über Leitung (3) die erste Kopffraktion enthaltend das polare Lösungsmittel und Leichtsieder, beispielsweise Wasser oder Methanol, entfernt werden. Über den Sumpf der ersten Destillationseinrichtung (2) wird mit der Leitung (4) die Sumpffraktion abgeführt. Bei der ersten Destillationseinrichtung handelt es sich um eine übliche Kolonne mit beispielsweise 2 bis 40 theoretischen Böden. Wird eine wässrige oder alkoholische Lösung aufgearbeitet, wird die erste Destillationseinrichtung so betrieben, dass in der über Leitung (4) abgeführten Sumpffraktion der Wasser- oder Alkoholgehalt maximal bis 5000 Gew.-ppm, bezogen auf die Masse der Sumpffraktion, beträgt. Die Sumpffraktion aus der ersten Destillationseinrichtung (2) wird auf eine zweite Destillationseinrichtung (5) gegeben, die mindestens 5 theoretische Böden aufweist und die als Dünnschichtverdampfer mit Kolonnenaufsatz ausgestaltet ist. Über Leitung (6) wird die zweite Kopffraktion entnommen, die beispielsweise Trimethylolpropan, Zwischenläufe, wie die unter Zwischenlauf I zusammengefassten zwischensiedenden Verbindungen, und Reste von dem polaren Lösungsmittel und von Leichtsiedern enthält. Falls der Gehalt an Trimethylolpropan in der zweiten Kopffraktion ausreichend hoch ist, kann dieser Produktstrom zweckmäßigerweise in den Prozess zur Herstellung von Trimethylolpropan zurückgeführt werden. Die Sumpffraktion aus der zweiten Destillationseinrichtung (5) wird über die Leitung (7) abgeführt und auf die dritte Destillationseinrichtung (8) gegeben. Diese dritte Destillationseinrichtung oder auch Nachlaufabtrenneinrichtung hat mindestens 4, beispielsweise 5 theoretische Böden und ist als Dünnschichtverdampfer mit Kolonnenaufsatz ausgestaltet. Über Leitung (9) wird Di-Trimethylolpropan als Kopffraktion in hoher Qualität abgeführt während über Leitung (10) Hochsieder aus dem Verfahren ausgeschleust werden.

Das erfindungsgemäße Verfahren gestattet die destillative Aufarbeitung von Di-Trimethylolpropan enthaltenden Lösungen und die wirksame Abtrennung von hochsiedenen Verunreinigungen mit zu Di-Trimethylolpropan vergleichbaren Siedepunkten, wie die unter Zwischenläufe I und II zusammengefassten Verbindungen. Di-Trimethylolpropan kann im Allgemeinen mit einem hohen Wertproduktgehalt von größer als 98 Gew.-%, gaschromatographisch ermittelt, erhalten werden. Auch können Sulfataschegehalte, bestimmt gemäß DIN 51575, modifiziert, von weniger als 100 ppm erreicht werden.

Werden in dem erfindungsgemäßen Verfahren als Di-Trimethyolpropan enthaltende Lösungen die Rückstände aus der Trimethylolpropanherstellung eingesetzt, so lassen sich gleichzeitig trimethylolpropanangereicherte Produktströme abtrennen und in den Herstellprozess für Trimethylolpropan zurückführen, so dass insgesamt auch die Anlageneffizienz und die Ausbeute an Trimethylolpropan verbessert werden kann.

In den nachfolgenden Beispielen wird das erfindungsgemäße Verfahren näher beschrieben. Es ist selbstverständlich nicht auf die wiedergegebene Ausführungsform beschränkt.

### Beispiel 1

Für die destillative Aufarbeitung kam eine wässrige Lösung zum Einsatz, die 40 Gew.-% Wasser und 60 Gew.-% organische Anteile enthielt, wobei der organische Anteil folgende gaschromatographisch ermittelte Zusammensetzung (%) aufwies:

| | |
|---|---|
| Vorlauf | 6,3 |
| Monocyclisches Formal (I) | 0,3 |
| Trimethylolpropan | 64,4 |
| Zwischenlauf I | 0,6 |
| Di-Trimethylolpropan | 28,0 |
| Zwischenlauf II | 0,1 |
| Lineares bis-Trimethylolpropan-Formal (II) | 0,1 |
| Hochsieder | 0,2 |

### Beispiel 1 a (1): Wasser-, Vorlaufentfernung

In einer ersten Destillation wurden an einer 20-Böden-Kolonne mit Sumpfblase bei einer Sumpftemperatur von 96°C und bei einem Druck von 73 hPa Wasser und Leichtsieder als Destillat entfernt. Der erhaltene Destillationssumpf enthielt etwa 800 Gew.-ppm Wasser und wies folgende gaschromatographisch ermittelte Zusammensetzung (%) auf:

| | |
|---|---|
| Vorlauf | 0,6 |
| Monocyclisches Formal (I) | 0,1 |
| Trimethylolpropan | 72,0 |
| Zwischenlauf I | 0,8 |
| Di-Trimethylolpropan | 26,0 |
| Zwischenlauf II | 0,1 |
| Lineares bis-Trimethylolpropan-Formal (II) | 0,1 |
| Hochsieder | 0,3 |

Beispiel 1 a (2): Leichtsiederentfernung, Trimethylolpropanabreicherung Das Sumpfprodukt gemäß Beispiel 1 a (1) wurde einer erneuten Destillation an einer 20-Böden-Kolonne mit Sumpfblase unterzogen. Der angelegte Druck betrug 3 hPa bei einer Sumpftemperatur von 255°C. Es wurde ein Rücklaufverhältnis von 1:1 eingestellt. Das erhaltene Sumpfprodukt wies folgende gaschromatographisch ermittelte Zusammensetzung (%) auf:

| | |
|---|---|
| Vorlauf | 0,1 |
| Monocyclisches Formal (I) | 0,0 |
| Trimethylolpropan | 2,4 |
| Zwischenlauf I | 1,6 |
| Di-Trimethylolpropan | 93,4 |
| Zwischenlauf II | 0,8 |
| Lineares bis-Trimethylolpropan-Formal (II) | 0,0 |
| Hochsieder | 1,7 |

### Beispiel 1b: Entfernung von Trimethylolpropan angereicherten Produktströmen

Das Sumpfprodukt aus der ersten Destillation gemäß Beispiel 1a (1) (70/30-Mischung) wurde für die zweite Destillation eingesetzt. Die zweite Destillation wurde so ausgestaltet, dass Zwischenlauffraktionen I und 11, die einen dem Di-Trimethylolpropan vergleichbaren Siedepunkt aufweisen, im Destillationssumpf möglichst abgereichert waren. In der Tabelle 1 sind unterschiedliche Ausführungsformen für die zweite Destillation zusammengestellt. Die gaschromatographische Analyse gibt die Zusammensetzung (%) des Einsatzes sowie die Zusammensetzung des Destillationssumpfes wieder.

**Tabelle 1: Entfernung von mit Trimethylolpropan angereicherten ProduktStrömen aus dem Destillationssumpf gemäß Beispiel 1 a (1) [Einsatz: 70/30-Mischung]; gaschromatographische Analyse der Sumpfprodukte**

| | | **1b (1)** | **1b (2)** | **1b (3) Vergleich** |
|---|---|---|---|---|
| | Einsatz (70/30-Mischung) | Dünnschichtverdampfer mit Füllkörperkolonne | Dünnschichtverdampfer mit Packungskolonne | Nur Packungskolonne mit Sumpfblase |
| Temperaturen | | | | |
| Kopf | [°C] | 160 | 163 | 165 |
| Seite | [°C] | - | 242 | 190 |
| Mantel / Sumpf | [°C] | 270 | 265 | 269 |
| Kolonnenkopf | [hPa] | 5 | 5 | 4 |
| Differenzdruck | [hPa] | 29 | 11 | 17 |
| Rücklaufverhältnis | | 1/3 | ohne | ohne |
| Kopfabnahme | [%] | 76 | 75,3 | 77 |
| Sumpfabnahme | [%] | 24 | 24,7 | 23 |
| Bodenzahl | | 11 | 15 | 15 |
| Verweilzeit | [s] | 10-30 | 10-30 | 3-5 Stunden |
| | | | | |

| Gaschromatographische Zusammensetzung (%): | | | | |
|---|---|---|---|---|
| Vorlauf | 0,6 | 0,1 | 0,1 | 0,1 |
| Monocyclisches Formal (I) | 0,1 | 0,0 | 0,0 | 0,0 |
| Trimethylolpropan | 72,0 | 0,1 | 0,3 | 0,1 |
| Zwischenlauf I | 0,8 | 1,5 | 0,4 | 0,9 |
| Di-Trimethylolpropan | 26,0 | 97,3 | 98,1 | 97,5 |
| Zwischenlauf II | 0,1 | 0,1 | 0,1 | 0,1 |
| Lineares bis-Trimethylol-propan -Formal (II) | 0,1 | 0,1 | 0,0 | 0,1 |
| Hochsieder | 0,3 | 0,8 | 1,0 | 1,2 |

Wie das Beispiel 1 b (2) zeigt, kann durch den Einsatz einer Packungskolonne, beispielsweise durch eine Kolonne mit einem Durchmesser von 40 mm und ausgestattet mit einer Montz-Packung der Zwischenlauf I im Sumpf abgereichert werden. Bei Beispiel 1 b (3) ist mit vergleichsweise hohen Verweilzeiten zu arbeiten, so dass es bei den hohen Destillationstemperaturen zu Zersetzungen unter Bildung flüchtiger Verbindungen kommt und ein vergleichsweise hoher Differenzdruck gegenüber Beispiel 1 b (2), das mit dem gleichen Kolonnentyp arbeitet, während der Destillation beobachtet wird. Dennoch wird auch bei dieser Ausgestaltung der zweiten Destillation ein Sumpfprodukt mit einem Gehalt von Di-Trimethylolpropan von 97,5% erhalten.

Der Einsatz von einer Packungskolonne mit einer höheren Anzahl an Trennstufen ist aber einer Füllkörperkolonne vorzuziehen.

Für die folgenden Destillationsversuche 1 b (4) - 1 b (6) wurde das Sumpfprodukt aus der Leichtsiederabtrennung und Trimethylolpropanabreicherung gemäß Beispiel 1 a (2) (93%ige Ware) verwendet. Die Bedingungen der zweiten Destillation sowie die gaschromatographische Analyse der Sumpfprodukte (%) sind in der folgenden Tabelle 2 zusammengestellt.

**Tabelle 2: Entfernung von mit Trimethylolpropan angereicherten Produktströmen aus dem Sumpfprodukt gemäß Beispiel 1a (2) (93%ige Ware, Einsatz) gaschromatographische Analyse der Sumpfprodukte**

| | | **1b (4)** | **1b (5) Vergleich** | **1b (6) Vergleich** |
|---|---|---|---|---|
| | Einsatz (93%ige Ware) | Dünnschichtverdampfer mit Packungskolonne | Nur Füllkörperkolonne mit Sumpfblase | Nur Kurzwegverdampfer ohne Kolonne |
| Temperaturen | | | | |
| Kopf | [°C] | 145-175 | 144-215 | 185-190 |
| Seite | [°C] | 149-180 | 148-223 | - |
| Mantel/Sumpf | [°C] | 260 | 270 | 200 |
| Kolonnenkopf | [hPa] | 1-2 | 4 | 3 |
| Differenzdruck | [hPa] | 8 | 16 | - |
| Rücklaufverhältnis | | ½ | 2/1 | ohne |
| Kopfabnahme | [%] | 24,3 | 13,1 | 50 |
| Sumpfabnahme | [%] | 75,7 | 86,9 | 50 |
| Bodenzahl | | 5 | 15 | 1 |
| Verweilzeit | [s] | 10-30 | 3-5 Stunden | 5-20 |
| | | | | |

| Gaschromatographische Zusammensetzung (%): | | | | |
|---|---|---|---|---|
| Vorlauf | 0,1 | 0,1 | 0,1 | 0,0 |
| Monocyclisches Formal (I) | 0,0 | 0,0 | 0,0 | 0,0 |
| Trimethylolpropan | 2,4 | 0,3 | 0,1 | 2,3 |
| Zwischenlauf I | 1,6 | 0,7 | 0,7 | 2,3 |
| Di-Trimethylolpropan | 93,4 | 97,7 | 96,7 | 94,1 |
| Zwischenlauf II | 0,8 | 0,1 | 0,1 | 0,2 |
| Lineares bis-Trimethylolpropan -Formal (II) | 0,0 | 0,0 | 0,0 | 0,0 |
| Hochsieder | 1,7 | 1,1 | 2,3 | 1,1 |

Wie das Vergleichsbeispiel 1 b (6) zeigt, gelingt die Abreicherung des Zwischenlaufs I im Sumpfprodukt der zweiten Destillation nicht, wenn ohne Kolonnenaufsatz und nur mit einem Kurzwegverdampfer gearbeitet wird. Für die Abreicherung des Zwischenlaufs I ist gemäß Beispiel 1 b (4) ein Kolonnenaufsatz mit 5 theoretischen Böden notwendig, selbst wenn der Destillationseinsatz bereits einen hohen Gehalt an Di-Trimethylolpropan aufweist.

### Beispiel 1 c: Nachlaufabtrennung

Das gemäß Beispiel 1 b (3) erhaltene Sumpfprodukt wurde für die dritte Destillation zur Nachlaufabtrennung eingesetzt. Das gewünschte Di-Trimethylolpropan wurde als Kopfprodukt in ausreichender Qualität erhalten. Die Destillationsbedingungen sowie die gaschromatographische Analyse (%) des Destillats sind in der Tabelle 3 angegeben.

**Tabelle 3: Nachlaufabtrennung aus dem Sumpfprodukt gemäß Beispiel 1 b (3), gaschromatographische Analyse (%) des Destillats**

| | | **1c (7)** | **1c (8) Vergleich** | **1c (9)** |
|---|---|---|---|---|
| | Einsatz Beispiel 1b (3) | Dünnschichtverdampfer mit Packungskolonne | Nur Füllkörperkolonne mit Sumpfblase | Dünnschichtverdampfer mit Packungskolonne |
| Temperaturen | | | | |
| Kopf | [°C] | 222 | 235 | 155 |
| Seite | [°C] | 230 | 240 | 160 |
| Sumpf | [°C] | 265 | 290-70 | 265 |
| Kolonnenkopf | [hPa] | 3 | 5 | 0,3 |
| Differenzdruck | [hPa] | 10 | 30-35 | - |
| Rücklaufverhältnis | | ohne | ohne | ohne |
| Kopfabnahme | [%] | 90,1 | 76,2 | 95,4 |
| Sumpfabnahme | [%] | 9,9 | 23,8 | 4,6 |
| Bodenzahl | | 15 | 15 | 15 |
| Verweilzeit | [s] | 5-8 | 3-5 Stunden | 5-9 |
| | | | | |

| Gaschromatographische Zusammensetzung (%): | | | | |
|---|---|---|---|---|
| Vorlauf | 0,1 | 0,0 | 11,5 | 0,0 |
| Monocyclisches Formal (I) | 0,0 | 0,0 | 0,0 | 0,0 |
| Trimethylolpropan | 0,1 | 0,6 | 14,8 | 0,5 |
| Zwischenlauf I | 0,9 | 0,1 | 2,2 | 0,1 |
| Di-Trimethylolpropan | 97,5 | 98,6 | 70,7 | 98,5 |
| Zwischenlauf II | 0,1 | 0,5 | 0,3 | 0,7 |
| Lineares bis-Trimethylolpropan -Formal (II) | 0,1 | 0,0 | 0,1 | 0,1 |
| Hochsieder | 1,2 | 0,2 | 0,4 | 0,1 |
| | | | | |
| Aschewert DIN 51575 modifiziert | | <50 ppm | - | <50 ppm |
| Gardner Farbzahl ASTM D1544 | >6 | 1 | 1 | 1 |

Wie der Vergleich der Beispiele 1 c (7) und 1 c (9) mit Vergleichsbeispiel 1 c (8) zeigt, ist die Verwendung eines Dünnschichtverdampfers mit Kolonnenaufsatz erforderlich um Di-Trimethylolpropan als Kopfprodukt in ausreichender Qualität zu erhalten. Eine Destillationseinrichtung aus Sumpfblase mit Kolonnenaufsatz ist für die Nachlaufabtrennung nicht geeignet, da es bei dieser Anordnung aufgrund der hohen Temperaturen und langen Verweilzeiten zu einer vermehrten Zersetzung, angezeigt durch eine deutliche Vorlaufbildung und Abnahme des Di-Trimethylolpropangehaltes, kommt.

## Patentansprüche

1. Destillatives Verfahren zur Gewinnung von Di-Trimethylolpropan aus Di-Trimethylolpropan enthaltenden Lösungen, **dadurch gekennzeichnet, dass** man:
(a) die Di-Trimethylolpropan enthaltende Lösung in einer ersten Destillationseinrichtung in eine erste Kopffraktion enthaltend leichtsiedende, niedriger als Di-Trimethylolpropan siedende Verbindungen und in eine Sumpffraktion auftrennt;
(b) die nach Schritt a) erhaltene Sumpffraktion auf eine zweite Destillationseinrichtung mit mindestens 5 theoretischen Böden, die als Dünnschichtverdampfer mit Kolonnenaufsatz ausgestaltet ist, gibt und eine zweite Kopffraktion enthaltend zwischensiedende, niedriger als Di-Trimethylolpropan siedende Verbindungen abzieht und eine Sumpffraktion entnimmt; und
(c) die nach Schritt b) erhaltene Sumpffraktion auf eine dritte Destillationseinrichtung mit mindestens 4 theoretischen Böden, die als Dünnschichtverdampfer mit Kolonnenaufsatz ausgestaltet ist, gibt, in der Di-Trimethylolpropan als dritte Kopffraktion gewonnen wird und Hochsieder als Sumpffraktion entfernt werden.

2. Verfahren gemäß Anspruch 1, **dadurch gekennzeichnet, dass** die zweite Destillationseinrichtung mindestens 8 theoretische Böden aufweist.

3. Verfahren gemäß Anspruch 1 oder 2, **dadurch gekennzeichnet, dass** in Schritt b) der Kolonnenaufsatz als Füllkörperkolonne oder Packungskolonne ausgestaltet ist.

4. Verfahren gemäß einem oder mehreren der Ansprüche 1 bis 3,
**dadurch gekennzeichnet, dass** die zweite Destillationseinrichtung bei einer Temperatur von 210 bis 280°C und bei einem Druck von 2 bis 10 hPa betrieben wird.

5. Verfahren gemäß einem oder mehreren der Ansprüche 1 bis 4,
**dadurch gekennzeichnet, dass** die zweite Destillationseinrichtung bei einer Verweilzeit von 2 bis 60 Sekunden betrieben wird.

6. Verfahren gemäß einem oder mehreren der Ansprüche 1 bis 5,
**dadurch gekennzeichnet, dass** die dritte Destillationseinrichtung 4 bis 20 theoretische Böden aufweist.

7. Verfahren gemäß einem oder mehreren der Ansprüche 1 bis 6,
**dadurch gekennzeichnet, dass** in Schritt c) der Kolonnenaufsatz als Füllkörperkolonne oder Packungskolonne ausgestaltet ist.

8. Verfahren gemäß einem oder mehreren der Ansprüche 1 bis 7,
**dadurch gekennzeichnet, dass** die dritte Destillationseinrichtung bei einer Temperatur von 240 bis 280°C und bei einem Druck von 0,2 bis 5 hPa betrieben wird.

9. Verfahren gemäß einem oder mehreren der Ansprüche 1 bis 8,
**dadurch gekennzeichnet, dass** in der dritten Destillationseinrichtung die Verweilzeit der Kopffraktion 1 bis 30 Sekunden beträgt.

10. Verfahren gemäß einem oder mehreren der Ansprüche 1 bis 9,
**dadurch gekennzeichnet, dass** eine Lösung von Di-Trimethylolpropan in einem polaren Lösungsmittel verwendet wird.

11. Verfahren gemäß Anspruch 10, **dadurch gekennzeichnet, dass** der Gehalt an dem polaren Lösungsmittel in der Sumpffraktion der ersten Destillationseinrichtung bis 5000 Gew.-ppm, bezogen auf die Sumpffraktion beträgt.

12. Verfahren gemäß Anspruch 11, **dadurch gekennzeichnet, dass** der Gehalt an dem polaren Lösungsmittel in der Sumpffraktion der ersten Destillationskolonne bis 1000 Gew.-ppm, insbesondere bis 500 Gew.-ppm, bezogen auf die Sumpffraktion beträgt.

13. Verfahren gemäß einem oder mehreren der Ansprüche 10 bis 13, **dadurch gekennzeichnet, dass** man als polares Lösungsmittel einen C₁-C₅-aliphatischen Alkohol, einen C₂-C₁₀-Dialkylether oder Wasser verwendet.

14. Verfahren gemäß einem oder mehreren der Ansprüche 10 bis 13, **dadurch gekennzeichnet, dass** die Di-Trimethylolpropan enthaltende Lösung aus den Rückständen der Trimethylolpropanherstellung unter Wasserzugabe hergestellt wird.

15. Verfahren gemäß einem oder mehreren der Ansprüche 1 bis 14, **dadurch gekennzeichnet, dass** man in dem destillativen Verfahren zur Gewinnung von Di-Trimethylolpropan eine Di-Trimethylolpropan enthaltende Lösung einsetzt, die mit einem Ionenaustauscher behandelt wurde.

16. Verfahren gemäß Anspruch 15, **dadurch gekennzeichnet, dass** die Ionenaustauscherbehandlung sowohl mit einem basischen als auch mit einem sauren Ionenaustauscher in beliebiger Reihenfolge erfolgt.

## Claims

1. Distillative process for obtaining ditrimethylolpropane from solutions comprising ditrimethylolpropane, **characterized in that**:
(a) the solution comprising ditrimethylolpropane is separated in a first distillation unit into a first tops fraction comprising low-boiling compounds having a lower boiling point than ditrimethylolpropane and a bottoms fraction;
(b) the bottoms fraction obtained after step a) is introduced into a second distillation unit having at least 5 theoretical plates, said unit being configured as a thin-film evaporator with a column attachment, and a second tops fraction comprising intermediate-boiling compounds having a lower boiling point than ditrimethylolpropane is drawn off, and a bottoms fraction is withdrawn; and
(c) the bottoms fraction obtained after step b) is introduced into a third distillation unit having at least 4 theoretical plates, said unit being configured as a thin-film evaporator with a column attachment, in which ditrimethylolpropane is obtained as a third tops fraction and high boilers are removed as a bottoms fraction.

2. Process according to Claim 1, **characterized in that** the second distillation unit has at least 8 theoretical plates.

3. Process according to Claim 1 or 2, **characterized in that**, in step b) the column attachment is configured as a column with random packing or column with structured packing.

4. Process according to one or more of Claims 1 to 3, **characterized in that** the second distillation unit is operated at a temperature of 210 to 280°C and at a pressure of 2 to 10 hPa.

5. Process according to one or more of Claims 1 to 4, **characterized in that** the second distillation unit is operated with a residence time of 2 to 60 seconds.

6. Process according to one or more of Claims 1 to 5, **characterized in that** the third distillation unit has 4 to 20 theoretical plates.

7. Process according to one or more of Claims 1 to 6, **characterized in that**, in step c) the column attachment is configured as a column with random packing or column with structured packing.

8. Process according to one or more of Claims 1 to 7, **characterized in that** the third distillation unit is operated at a temperature of 240 to 280°C and at a pressure of 0.2 to 5 hPa.

9. Process according to one or more of Claims 1 to 8, **characterized in that** the residence time of the tops fraction in the third distillation unit is 1 to 30 seconds.

10. Process according to one or more of Claims 1 to 9, **characterized in that** a solution of ditrimethylolpropane in a polar solvent is used.

11. Process according to Claim 10, **characterized in that** the content of the polar solvent in the bottoms fraction of the first distillation unit is up to 5000 ppm by weight, based on the bottoms fraction.

12. Process according to Claim 11, **characterized in that** the content of the polar solvent in the bottoms fraction of the first distillation column is up to 1000 ppm by weight, especially up to 500 ppm by weight, based on the bottoms fraction.

13. Process according to one or more of Claims 10 to 13, **characterized in that** the polar solvent used is a C₁-C₅ aliphatic alcohol, a C₂-C₁₀ dialkyl ether or water.

14. Process according to one or more of Claims 10 to 13, **characterized in that** the solution comprising ditrimethylolpropane is produced from the residues of trimethylolpropane preparation with addition of water.

15. Process according to one or more of Claims 1 to 14, **characterized in that** a solution which comprises ditrimethylolpropane and has been treated with an ion exchanger is used in the distillative process for obtaining ditrimethylolpropane.

16. Process according to Claim 15, **characterized in that** the ion exchanger treatment is effected both with a basic and with an acidic ion exchanger in any sequence.

## Revendications

1. Procédé de distillation pour la production de di-triméthylolpropane à partir de solutions contenant du di-triméthylolpropane, **caractérisé en ce qu'**on
(a) sépare la solution contenant du di-triméthylolpropane, dans un premier dispositif de distillation, en une première fraction de tête, contenant des composés à bas point d'ébullition, bouillant sous le di-triméthylolpropane, et en une fraction de fond ;
(b) introduit la fraction de fond obtenue selon l'étape a) sur un deuxième dispositif de distillation présentant au moins 5 plateaux théoriques, qui est conçu comme évaporateur à couches minces présentant un adaptateur de colonne, et on soutire une deuxième fraction de tête contenant des composés à point d'ébullition intermédiaire, bouillant sous le di-triméthylolpropane, et on prélève une fraction de fond ; et
(c) introduit la fraction de fond obtenue selon l'étape b) sur un troisième dispositif de distillation présentant au moins 4 plateaux théoriques, qui est conçu comme évaporateur à couches minces présentant un adaptateur de colonne, dans lequel on obtient le di-triméthylolpropane comme troisième fraction de tête et des substances à point d'ébullition élevé sont éliminées en tant que fraction de fond.

2. Procédé selon la revendication 1, **caractérisé en ce que** le deuxième dispositif de distillation présente au moins 8 plateaux théoriques.

3. Procédé selon la revendication 1 ou 2, **caractérisé en ce que**, dans l'étape b), l'adaptateur de colonne est conçu comme une colonne à corps de remplissage ou comme une colonne garnie.

4. Procédé selon l'une ou plusieurs des revendications 1 à 3, **caractérisé en ce que** le deuxième dispositif de distillation est exploité à une température de 210 à 280°C et à une pression de 2 à 10 hPa.

5. Procédé selon l'une ou plusieurs des revendications 1 à 4, **caractérisé en ce que** le deuxième dispositif de distillation est exploité à un temps de séjour de 2 à 60 secondes.

6. Procédé selon l'une ou plusieurs des revendications 1 à 5, **caractérisé en ce que** le troisième dispositif de distillation présente 4 à 20 plateaux théoriques.

7. Procédé selon l'une ou plusieurs des revendications 1 à 6, **caractérisé en ce que**, dans l'étape c), l'adaptateur de colonne est conçu comme une colonne à corps de remplissage ou comme une colonne garnie.

8. Procédé selon l'une ou plusieurs des revendications 1 à 7, **caractérisé en ce que** le troisième dispositif de distillation est exploité à une température de 240 à 280°C et à une pression de 0,2 à 5 hPa.

9. Procédé selon l'une ou plusieurs des revendications 1 à 8, **caractérisé en ce que**, dans le troisième dispositif de distillation, le temps de séjour de la fraction de tête est de 1 à 30 secondes.

10. Procédé selon l'une ou plusieurs des revendications 1 à 9, **caractérisé en ce qu'**on utilise une solution de di-triméthylolpropane dans un solvant polaire.

11. Procédé selon la revendication 10, **caractérisé en ce que** la teneur en solvant polaire dans la fraction de fond du premier dispositif de distillation est de jusqu'à 5000 ppm en poids, par rapport à la fraction de fond.

12. Procédé selon la revendication 11, **caractérisé en ce que** la teneur en solvant polaire dans la fraction de fond de la première colonne de distillation est de jusqu'à 1000 ppm en poids, en particulier de jusqu'à 500 ppm en poids, par rapport à la fraction de fond.

13. Procédé selon l'une ou plusieurs des revendications 10 à 13, **caractérisé en ce qu'**on utilise, comme solvant polaire, un alcool aliphatique en C₁-C₅, un C₂-C₁₀-dialkyléther ou de l'eau.

14. Procédé selon l'une ou plusieurs des revendications 10 à 13, **caractérisé en ce que** la solution contenant du di-triméthylolpropane est préparée à partir des résidus de la préparation de triméthylolpropane avec addition d'eau.

15. Procédé selon l'une ou plusieurs des revendications 1 à 14, **caractérisé en ce qu'**on utilise, dans le procédé de distillation pour la production de di-triméthylolpropane, une solution contenant du di-triméthylolpropane qui a été traitée par un échangeur d'ions.

16. Procédé selon la revendication 15, **caractérisé en ce que** le traitement par un échangeur d'ions est réalisé tant avec un échangeur d'ions basique qu'avec un échangeur d'ions acide, dans un ordre quelconque.
